# FASCICULE DE BREVET EUROPEEN

(11) **EP 3 717 048 B1**
(45) Date de publication et mention de la délivrance du brevet: **18.08.2021**
(21) Numéro de dépôt: 18830906.6
(22) Date de dépôt: 26.11.2018
(51) Int. Cl.: A61M 15/00

(54) **DISPOSITIF DE DISTRIBUTION DE PRODUIT FLUIDE SYNCHRONISE AVEC L'INHALATION**
VORRICHTUNG ZUR INHALATIONSSYNCHRONISIERTEN AUSGABE EINES FLÜSSIGPRODUKTS
DEVICE FOR INHALATION-SYNCHRONISED DISPENSING OF A FLUID PRODUCT

(30) Priorité: 29.11.2017 FR 1761334
(43) Date de publication de la demande: 07.10.2020
(73) Titulaire: APTAR France SAS, 27110 Le Neubourg (FR)
(72) Inventeur: PETIT, Ludovic, 27110 Vitot (FR)
(74) Mandataire: CAPRI
(86) Numéro de dépôt international: PCT/FR2018/052979
(87) Numéro de publication internationale: WO 2019/106270

(56) Documents cités:
- WO-A1-2004/028608
- WO-A1-2009/044172
- WO-A1-2010/003846
- WO-A1-2017/178764
- WO-A1-2017/178765
- WO-A1-2017/178767
- US-A- 5 060 643

## Description

La présente invention concerne un dispositif de distribution de produit fluide synchronisé avec l'inhalation, et plus particulièrement un dispositif d'inhalation du type aérosol synchronisé avec l'inhalation.

Les dispositifs actionnés par l'inhalation, désignés généralement par le terme B.A.I. (signifiant "Breath Actuated Inhaler"), sont bien connus dans l'état de la technique. L'avantage principal de ce type de dispositif est que la distribution du produit est synchronisée avec l'inhalation du patient, pour garantir une bonne distribution du produit dans les voies respiratoires. Ainsi, dans le domaine des dispositifs aérosol, c'est-à-dire ceux dans lesquels le produit est distribué à l'aide d'un gaz propulseur, de nombreux types de dispositifs de déclenchement par l'inhalation ont été proposés. Ces dispositifs présentent toutefois l'inconvénient de comporter un grand nombre de pièces, c'est-à-dire qu'ils sont compliqués et coûteux à fabriquer et à assembler, ce qui est évidemment désavantageux. Il est aussi difficile de trouver le bon équilibre entre un déclenchement fiable à chaque inhalation, sans que le seuil de déclenchement ne soit trop élevé, et une serrure suffisamment robuste pour empêcher un actionnement accidentel ou non souhaité. Or, si la serrure se déverrouille de manière accidentelle, le dispositif est actionné automatiquement et la dose est distribuée, même si l'utilisateur ne l'a pas souhaité.

Ainsi, plus que l'actionnement automatique du dispositif, ce qui est important pour obtenir une bonne distribution de la dose, c'est de réaliser cette distribution de manière synchronisée avec l'inhalation de l'utilisateur, même si l'actionnement ou le déclenchement proprement dit reste manuel.

Les documents FR2775668, WO2017178764, WO2017178765 et WO2017178768 décrivent des dispositifs de l'état de la technique. Le document WO2017178764, en particulier, décrit un dispositif de distribution de produit fluide synchronisé avec l'inhalation, comportant un corps pourvu d'un embout buccal, un réservoir de produit contenant un produit fluide et un gaz propulseur étant monté axialement coulissant par rapport audit corps, une valve doseuse comportant une soupape étant assemblée sur ledit réservoir pour distribuer sélectivement le produit fluide. Le dispositif comporte également: un élément de blocage déplaçable et/ou déformable entre une position de blocage, dans laquelle ladite valve doseuse ne peut pas être actionnée, et une position d'actionnement, dans laquelle ladite valve doseuse peut être actionnée; un élément déclencheur déplaçable et/ou déformable entre une position de verrouillage, dans laquelle il bloque ledit élément de blocage dans sa position de blocage, et une position de libération, dans laquelle il ne bloque pas ledit élément de blocage; et un système de déclenchement commandé par l'inhalation comportant un organe sensible à l'inhalation, déformable et/ou déplaçable sous l'effet de l'inhalation, ledit organe sensible à l'inhalation coopérant avec ledit élément déclencheur, de sorte que lorsque ledit organe sensible à l'inhalation se déforme et/ou se déplace, il déplace et/ou déforme ledit élément déclencheur vers sa position de libération, permettant ainsi le déplacement et/ou la déformation dudit élément de blocage de sa position de blocage vers sa position d'actionnement. Un organe d'actionnement est également assemblé sur le réservoir du côté axialement opposé à ladite valve doseuse, ledit organe d'actionnement comportant un manchon creux déplaçable axialement par rapport audit réservoir entre une position de repos et une position chargée, un ressort étant disposé entre le fond du réservoir et le bord supérieur fermé dudit manchon creux, de sorte que lorsque l'utilisateur appuie manuellement sur ledit organe d'actionnement pour le déplacer vers sa position chargée, ledit ressort est comprimé, pour transmettre une force axiale audit réservoir. L'organe d'actionnement comporte une patte de blocage, coopérant en position de repos avec ledit élément déclencheur pour l'empêcher de se déplacer vers sa position de libération.

Un inconvénient de ces dispositifs réside dans les risques de blocage si l'effort d'actionnement de l'utilisateur est trop important, c'est-à-dire s'il exerce sur le fond du réservoir une force axiale d'actionnement trop forte.

La présente invention a pour but de fournir un dispositif de distribution de produit fluide synchronisé avec l'inhalation qui ne reproduit pas les inconvénients susmentionnés.

La présente invention a aussi pour but de fournir un dispositif de distribution de produit fluide synchronisé avec l'inhalation qui améliore la fiabilité de fonctionnement, en garantissant un actionnement efficace à chaque inhalation, sans risque de blocage.

La présente invention a aussi pour but de fournir un dispositif de distribution de produit fluide synchronisé avec l'inhalation qui minimise les risques d'actionnement accidentel ou non souhaité.

La présente invention a également pour but de fournir un dispositif de distribution de produit fluide synchronisé avec l'inhalation qui ne présente pas un seuil de déclenchement trop élevé, permettant ainsi à des personnes relativement faibles, telles que des personnes malades ou âgées, d'utiliser le dispositif de manière sûre et fiable.

La présente invention a également pour but de fournir un dispositif de distribution de produit fluide synchronisé avec l'inhalation qui soit simple et peu coûteux à fabriquer et à assembler.

La présente a donc pour objet un dispositif de distribution de produit fluide synchronisé avec l'inhalation, comportant un corps pourvu d'un embout buccal, un réservoir de produit contenant un produit fluide et un gaz propulseur étant monté axialement coulissant dans ledit corps, une valve doseuse comportant une soupape étant assemblée sur ledit réservoir pour distribuer sélectivement le produit fluide, ledit dispositif comportant :
- un élément de blocage déplaçable et/ou déformable entre une position de non actionnement, dans laquelle ladite valve doseuse ne peut pas être actionnée, et une position d'actionnement, dans laquelle ladite valve doseuse peut être actionnée, ledit élément de blocage, en position de non actionnement, coopérant d'une part avec le corps et d'autre part avec le réservoir pour empêcher le déplacement axial dudit réservoir dans le corps, et
- un système de déclenchement commandé par l'inhalation comportant un organe sensible à l'inhalation déformable et/ou déplaçable sous l'effet de l'inhalation, ledit organe sensible à l'inhalation, lorsqu'il se déforme et/ou se déplace, déplaçant et/ou déformant ledit élément de blocage de sa position de non actionnement vers sa position d'actionnement,
ledit dispositif comportant un actionneur fixé sur ledit réservoir, ledit actionneur comportant une paroi proximale fixée, notamment emmanché à force, sur le fond du réservoir, un corps coulissant axialement autour de ladite paroi proximale, et un ressort disposé entre ladite paroi proximale et une paroi distale dudit corps, ledit corps coulissant axialement autour de ladite paroi proximale entre une position de repos et une position chargée, dans laquelle ledit ressort est comprimé, de sorte qu'après actionnement dudit système de déclenchement commandé par l'inhalation, ledit ressort comprimé déplace axialement ledit réservoir dans ledit corps pour actionner ladite valve doseuse.

Avantageusement, ledit élément de blocage est une bague de blocage fixée, notamment encliquetée, sur ledit réservoir et comportant au moins une patte axiale, notamment trois, coopérant avec un épaulement solidaire dudit corps pour bloquer le déplacement axial dudit réservoir dans ledit corps.

Avantageusement, ladite au moins une patte axiale est déformable radialement vers l'extérieur pour passer de la position de non actionnement vers la position d'actionnement, un élément déclencheur étant prévu pour maintenir ladite au moins une patte axiale en position de non actionnement.

Avantageusement, ledit élément déclencheur est monté déplaçable entre une position de blocage, dans laquelle il bloque ledit élément de blocage dans sa position de non actionnement, et une position de libération, dans laquelle il ne bloque pas ledit élément de blocage.

Avantageusement, ledit organe sensible à l'inhalation dudit système de déclenchement commandé par l'inhalation comporte une membrane déformable définissant une chambre d'air déformable, ladite membrane déformable étant fixée audit élément déclencheur, ladite membrane déformable étant déformée lors de l'inhalation de telle sorte qu'elle déplace ledit élément déclencheur de sa position de blocage vers sa position de libération.

Avantageusement, ledit ressort est un ressort à boudin.

En variante, ledit ressort peut être un ressort plastique.

En variante, ledit ressort peut être un élément en mousse.

Avantageusement, ledit l'actionneur comporte une bride radiale s'étendant radialement vers l'extérieur de part et d'autre dudit corps.

Avantageusement, la position de repos dudit actionneur est définie par une butée axiale formée entre un bord axial inférieur de ladite paroi proximale et un épaulement radial interne dudit corps.

Avantageusement, la position chargée dudit actionneur est définie par une butée axiale formée entre un bord axial inférieur dudit corps et un bord axial supérieur d'un manchon interne solidaire dudit corps.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique en section transversale d'un dispositif de distribution de produit fluide, en position de repos,
- la figure 2 est une vue similaire à celle de la figure 1, en position de non distribution avant aspiration,
- la figure 3 est une vue agrandie du détail A de la figure 2,
- la figure 4 est une vue agrandie du détail B de la figure 2,
- la figure 5 est une vue similaire à celle de la figure 2, en position après aspiration,
- la figure 6 est une vue similaire à celle de la figure 5, en position de distribution d'une dose de produit fluide,
- la figure 7 est une vue de détail agrandie d'une partie de la figure 1,
- la figure 8 est une vue schématique en perspective de l'actionneur de la figure 1,
- la figure 9 est une vue schématique de dessus de l'actionneur de la figure 1,
- la figure 10 est une vue schématique en section transversale de l'actionneur de la figure 1, et
- la figure 11 est une vue similaire à celle de la figure 10, illustrant une variante de réalisation.

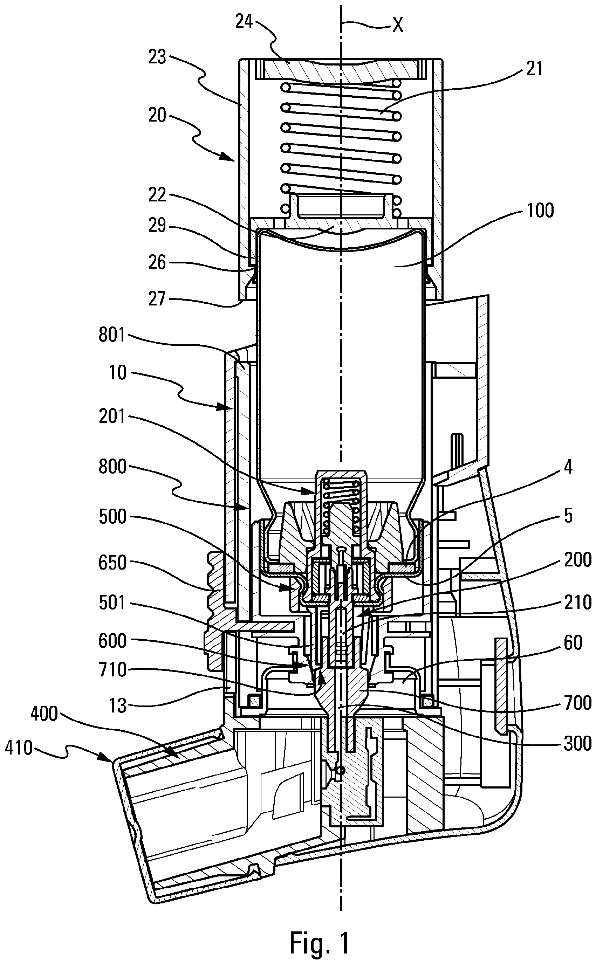

Dans la description, les termes "supérieur", "inférieur", "haut" et "bas" se réfèrent à la position du dispositif représentée notamment sur la figure 1. Les termes "axial" et "radial" se réfèrent à l'axe central vertical X représenté notamment sur la figure 1. Les termes "proximal" et "distal" se réfèrent à l'embout buccal.

L'invention s'applique plus particulièrement à des dispositifs d'inhalation du type à valve aérosol pour une distribution orale, comme cela sera décrit plus en détail ci-après, mais elle pourrait aussi s'appliquer à d'autres types de dispositifs d'inhalation, par exemple du type nasal.

Les figures représentent un mode de réalisation avantageux de l'invention, mais il est entendu que l'une ou plusieurs des pièces constitutives décrites ci-après pourrai(en)t être réalisée(s) différemment tout en procurant des fonctions similaires voire identiques.

En référence aux dessins, le dispositif comporte un corps principal 10 pourvu d'un embout buccal 400. Cet embout buccal 400 définit un orifice de distribution à travers lequel l'utilisateur va inhaler lors de l'utilisation du dispositif. Un capot de protection amovible 410 peut être prévu sur ledit embout buccal 400, notamment lors des périodes de stockage, que l'utilisateur va retirer avant utilisation. La figure 1 montre un tel capot de protection, qui pourrait être de forme quelconque.

Un manchon interne 800 peut être prévu à l'intérieur dudit corps 10, comme visible sur les figures.

Le corps 10 contient un réservoir 100, contenant le produit à distribuer et un gaz propulseur, tel qu'un gaz du type HFA, une valve doseuse 200 étant montée sur ledit réservoir 100 pour distribuer sélectivement le produit. La valve doseuse 200 comporte un corps de valve 201 et une soupape 210 axialement déplaçable lors de l'actionnement par rapport audit corps de valve 201, et donc par rapport audit réservoir 100. Cette valve doseuse 200 peut être d'un type quelconque approprié. Elle peut être fixée au réservoir 100 par un élément de fixation, de préférence une capsule sertie 5, de préférence avec interposition d'un joint de col 4.

Avantageusement, lors de l'actionnement, la soupape 210 est fixe par rapport au corps 10, et c'est le réservoir 100 qui est déplacé axialement par rapport au corps 10 entre une position distale, qui est la position de repos, et une position proximale. Dans l'exemple des figures, le réservoir coulisse axialement à l'intérieur dudit manchon interne 800.

L'orifice de sortie de la soupape 210 de ladite valve doseuse 200 est relié via un canal 300 audit embout buccal 400, à travers lequel l'utilisateur inhale le produit distribué. De manière connue, ladite soupape 210 est reçue dans un puits de soupape 700 qui définit au moins partiellement ledit canal 300. Le puits de soupape 700 peut être formé d'une pièce avec le corps 10, ou, comme dans l'exemple représenté, être formé par une pièce assemblée sur ledit corps 10.

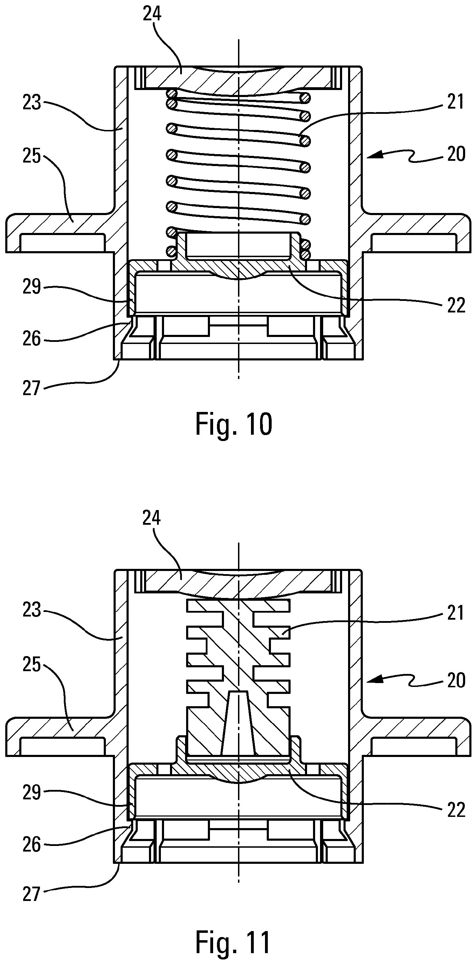

Selon l'invention, le dispositif comporte un actionneur 20. Cet actionneur 20 est monté, notamment emmanché à force, sur le fond du réservoir 100 et comporte un ressort 21 adapté à être comprimé lorsque l'utilisateur appuie axialement sur ledit actionneur 20. Ce ressort 21 peut être un ressort à boudin classique, comme représenté notamment sur la figure 10, ou en variante un ressort plastique ou un élément en mousse, comme visible sur la figure 11. Cet actionneur 20 permet de garantir une force d'actionnement de la valve 200 parfaitement maitrisée, reproductible et indépendante de l'effort d'actionnement réalisé par l'utilisateur. C'est toujours le ressort 21 comprimé qui déplace axialement le réservoir 100 pour actionner la valve 200. Ceci permet notamment d'éviter tout risque de blocage qui pourrait survenir en cas d'effort d'actionnement trop important par l'utilisateur.

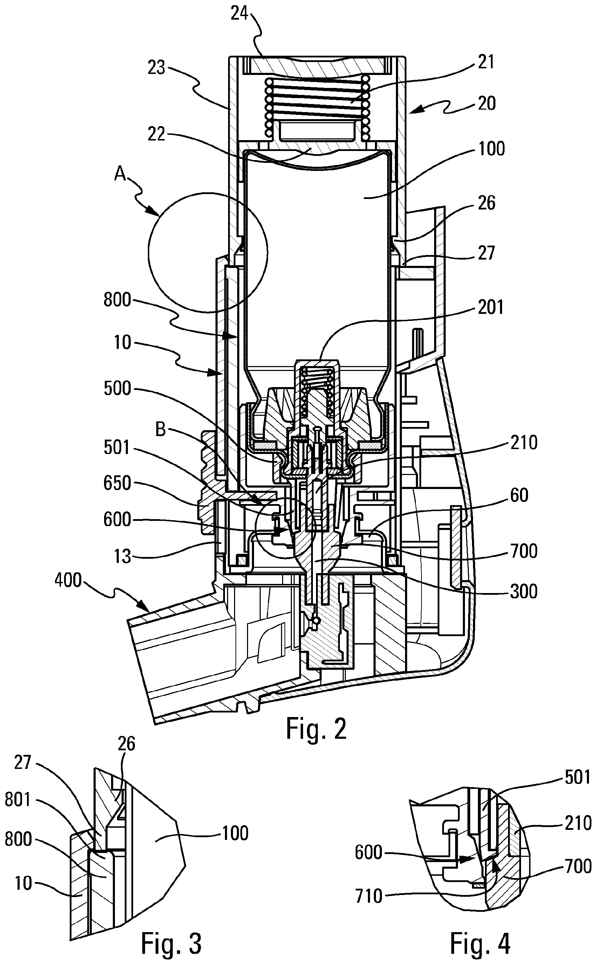

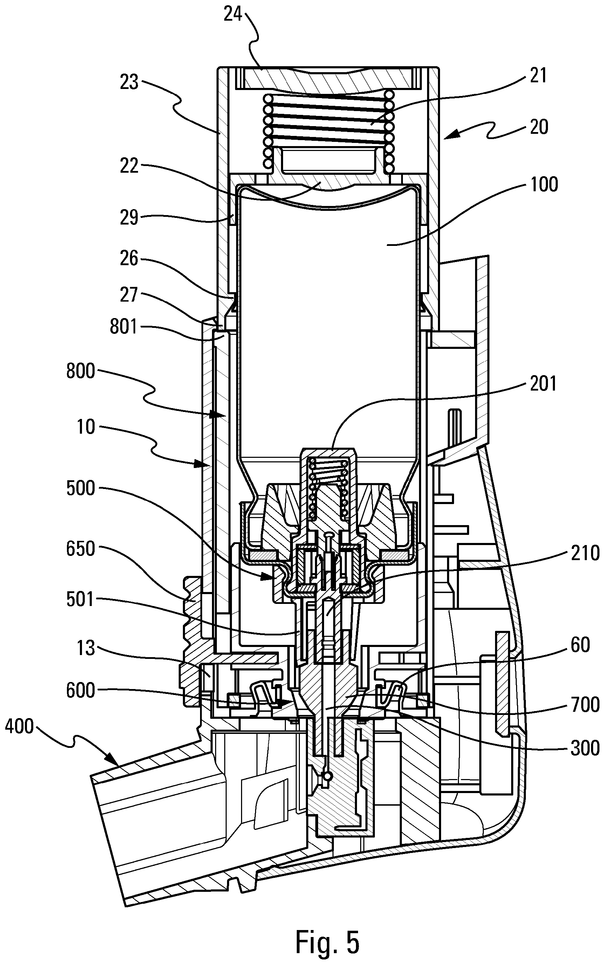

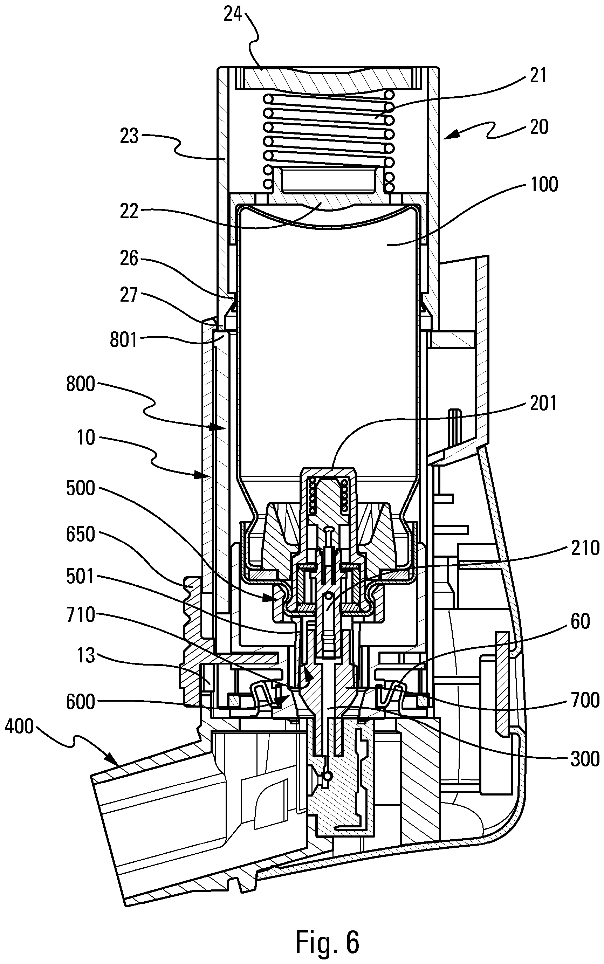

Comme visible notamment sur les figures 10 et 11, l'actionneur 20 comporte une paroi proximale 22 fixée, notamment emmanché à force, sur le fond du réservoir 100, et un corps 23 coulissant axialement autour de ladite paroi proximale 22, avec le ressort 21 disposé entre ladite paroi proximale 22 et une paroi distale 24 dudit corps 23. Ledit corps 23 coulisse axialement autour de ladite paroi proximale 22 d'une part entre une position de repos, visible sur les figures 1, 10 et 11, et une position chargée, visible sur les figures 2 et 5, dans laquelle le ressort 21 est comprimé au maximum. D'autre part, ledit corps 23 coulisse axialement autour de ladite paroi proximale 22 entre ladite position chargée et une position actionnée, visible sur la figure 6, dans laquelle le ressort 21 a transmis sa force pour déplacer le réservoir 100 dans le corps 10 pour actionner la valve 200.

Avantageusement, l'actionneur 20 peut comporter une bride radiale 25 s'étendant radialement vers l'extérieur de part et d'autre du corps 23, permettant un appui axial facilité pour l'utilisateur. Bien entendu, cette bride radiale 25 n'est pas obligatoire et l'appui axial de l'utilisateur peut aussi se faire sur ladite paroi distale 24 du corps 23.

Dans l'exemple représenté sur les figures, la position de repos de l'actionneur 20 est avantageusement définie par une butée axiale formée entre un bord axial inférieur 29 de ladite paroi proximale 22 et un épaulement radial interne 26 du corps 23. La position chargée est avantageusement définie par une butée axiale formée entre un bord axial inférieur 27 du corps 23 et un bord axial supérieur 801 du manchon interne 800. En variante, cette butée axiale de la position chargée pourrait aussi être formée avec un bord axial supérieur du corps 10.

Le dispositif comporte un élément de blocage 500 déplaçable et/ou déformable entre une position de non actionnement, dans laquelle ladite valve doseuse 200 ne peut pas être actionnée, et une position d'actionnement, dans laquelle ladite valve doseuse 200 peut être actionnée. En position de repos, ledit élément de blocage 500 est en position de non actionnement, et c'est l'inhalation de l'utilisateur à travers l'embout buccal 400 qui déplace et/ou déforme ledit élément de blocage 500 vers sa position d'actionnement. En d'autres termes, tant que l'utilisateur n'a pas inhalé, l'actionnement de la valve doseuse 200 est impossible, et ce n'est que lorsqu'il inhale que ladite valve doseuse 200 peut être actionnée.

Comme cela sera décrit plus en détails ci-après, l'élément de blocage 500, en position de non actionnement, empêche le déplacement axial du réservoir 100 dans le corps 10. Lors de l'inhalation, cet élément de blocage 500 est déplacé et/ou déformé de telle sorte qu'il ne bloque plus le déplacement axial du réservoir 100 dans le corps 10. Ainsi, après inhalation, un tel déplacement axial du réservoir 100 provoque l'actionnement de la valve doseuse 200 et la distribution d'une dose de produit synchronisée avec cette inhalation.

Ainsi, en l'absence d'inhalation, il n'y a aucun risque qu'une dose de produit actif soit perdue par un actionnement malencontreux ou incomplet dans lequel l'utilisateur n'exercerait pas d'inhalation. L'actionnement de la valve 200 et l'expulsion d'une dose de produit fluide ne sont donc possibles que si l'utilisateur inhale et que simultanément le réservoir 100 se déplace dans le corps 10 pour actionner la valve 200.

Le dispositif comporte un système de déclenchement commandé par l'inhalation de l'utilisateur, qui est destiné à déplacer et/ou déformer ledit élément de blocage 500 de sa position de non actionnement vers sa position d'actionnement, lorsque l'utilisateur inhale à travers l'embout buccal 400.

Ce système de déclenchement comporte un organe sensible à l'inhalation 60 déformable et/ou déplaçable sous l'effet de l'inhalation, cet organe sensible à l'inhalation 60 étant adapté, lorsqu'il se déforme et/ou se déplace, à déplacer et/ou déformer ledit élément de blocage 500 de sa position de non actionnement vers sa position d'actionnement.

Comme cela sera décrit plus en détails ci-après, l'organe sensible à l'inhalation 60 peut être réalisé sous la forme d'une chambre d'air déformable, par exemple un soufflet ou une poche déformable.

Avantageusement, la position de non actionnement correspond à une position de blocage du réservoir 100 dans le corps 10. Dans cette position de blocage, le réservoir 100 est empêché de se déplacer par ledit élément de blocage 500, qui ne sera libéré qu'au moment de l'inhalation.

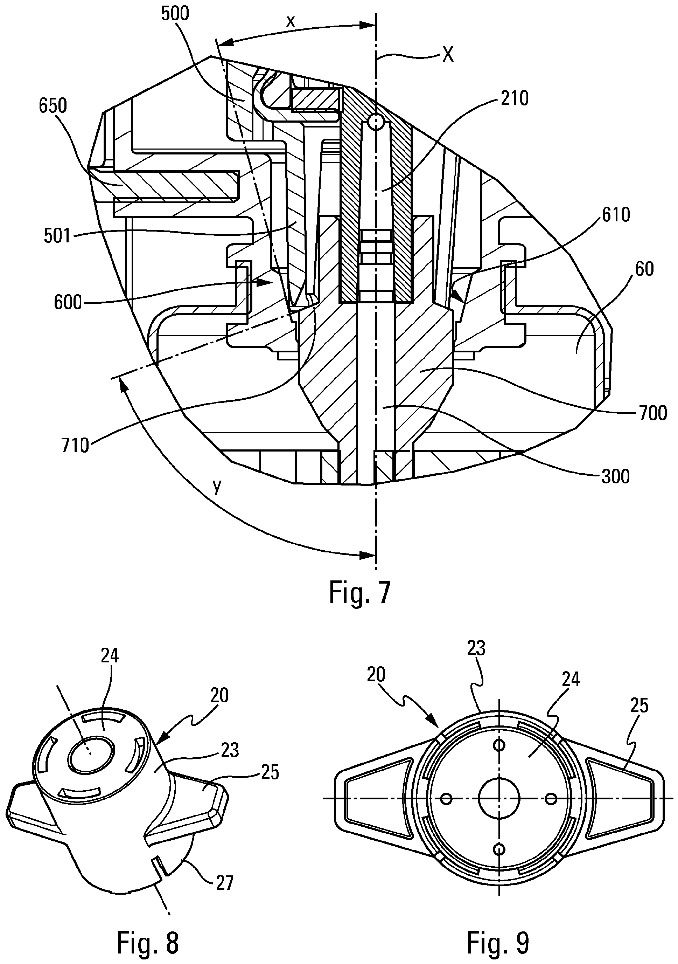

L'élément de blocage 500 est avantageusement formé par une bague de blocage, comportant au moins une, de préférence trois pattes axiales de blocage 501, qui sont élastiquement déformable radialement vers l'extérieur. Cette bague de blocage est fixée, notamment encliquetée, sur le réservoir 100, notamment sur la capsule 5 qui fixe la valve doseuse 200 sur le réservoir 100. Lesdites pattes de blocage 501 s'appuient en position de repos sur un épaulement radial 710 (mieux visible sur les figures 4 et 7) dudit puits de soupape 700. Cet épaulement radial 710 est de préférence incliné vers le bas et radialement vers l'extérieur, formant un angle y avec l'axe central longitudinal X de la valve 200, de sorte que lorsque le réservoir 100 coulisse axialement dans le corps 10 lors de l'actionnement, lesdites pattes axiales de blocage 501 glissent sur ledit épaulement 710 incliné, ce qui les déforme radialement vers l'extérieur. Avantageusement, cet angle y est supérieur à 20°, de préférence supérieur à 30°, et bien entendu inférieur à 90°.

Un élément déclencheur 600 est monté coulissant axialement autour dudit puits de soupape 700 entre une position de blocage, dans laquelle il bloque ledit élément de blocage 500 dans sa position de non actionnement, et une position de libération, dans laquelle il ne bloque plus ledit élément de blocage 500. En particulier, ledit élément déclencheur 600, en position de blocage, coopère avec lesdites pattes de blocage 501, empêchant toute déformation radiale vers l'extérieur desdites pattes de blocage 501. Ainsi, lorsque ledit élément déclencheur 600 est en position de blocage, il empêche la déformation radiale vers l'extérieur desdites pattes de blocage 501, qui par conséquent restent axialement bloquées par ledit épaulement 710 du puits de soupape 700, ce qui bloque le déplacement axial du réservoir 100 et donc l'actionnement de la valve doseuse 200. Avantageusement, la surface 610 de l'élément déclencheur 600, qui coopère avec lesdites pattes de blocage 501 en position de blocage, est inclinée et forme un angle x avec l'axe central longitudinal X de la valve 200, comme représenté sur la figure 7. Cet angle x est avantageusement inférieur à 30°, de préférence inférieur à 15°.

Selon les spécifications requises, on peut optimiser les angles x et y afin de faciliter l'actionnement du système de déclenchement commandé par l'inhalation.

L'élément déclencheur 600 est relié audit système de déclenchement commandé par l'inhalation, en particulier audit organe sensible à l'inhalation 60, de sorte que lorsque ce dernier se déforme et/ou se déplace, il fait passer ledit élément déclencheur 600 de sa position de blocage vers sa position de libération.

En position de blocage, l'élément déclencheur 600 coopère avantageusement de manière sensiblement étanche avec l'extérieur du puits de soupape 700, pour mobiliser la majeure partie du flux d'inhalation à l'actionnement du système de déclenchement commandé par l'inhalation. Lorsque l'élément déclencheur 600 quitte sa position de blocage et va vers sa position de libération, il ouvre un passage d'air autour dudit puits de soupape, comme visible sur les figures 5 et 6. Ceci favorise l'inhalation en générant un appel d'air, améliorant ainsi l'efficacité de la prise de dose. De même, cet appel d'air rend la processus d'inhalation plus confortable pour l'utilisateur.

L'élément déclencheur 600 est avantageusement fixé à un manchon externe 650 formant couvercle et qui coulisse axialement à l'extérieur du corps 10. Ce couvercle 650 coopère avec une ouverture 13 pour substantiellement ouvrir cette ouverture 13 dans la position de blocage, et pour substantiellement fermer cette ouverture 13 dans la position de libération. Cette ouverture 13 étant ouverte en début d'inhalation, elle permet un actionnement fiable du système de déclenchement par l'inhalation, en particulier l'organe sensible à l'inhalation 60, en évitant toute dépression à l'extérieur dudit organe sensible à l'inhalation 60 lorsqu'il se déforme. Ceci permet d'optimiser ce déclenchement par l'inhalation. Lorsque l'élément déclencheur 600 s'est déplacé axialement vers sa position de libération sous l'effet de l'inhalation, et donc lorsque la valve doseuse 200 peut être actionnée pour distribuer une dose de produit fluide, le couvercle 650 ferme l'ouverture 13.

Avantageusement, ledit couvercle 650 est accessible de l'extérieur. Ceci permet en cas de nécessité de déplacer manuellement l'élément déclencheur 600, pour pouvoir réaliser un actionnement de la valve doseuse 200 même sans inhalation, par exemple si la personne devant recevoir la dose de produit fluide est incapable de réaliser une inhalation suffisante. Il s'agit donc d'une sécurité.

L'organe sensible à l'inhalation 60 est avantageusement réalisé sous la forme d'une chambre d'air déformable. Avantageusement, cette chambre d'air comprend une membrane déformable qui est reliée d'une part au corps 10 et d'autre part audit élément déclencheur 600.

Lors de l'inhalation, la membrane déformable de l'organe sensible à l'inhalation 60 se déforme et/ou se contracte sous l'effet de la dépression générée par l'inhalation, provoquant le déplacement de l'élément déclencheur 600 de sa position de blocage vers sa position de libération. Ceci permet alors la déformation radiale desdites pattes de blocage 501 et donc le déplacement dudit élément de blocage 500 de sa position de non actionnement vers sa position d'actionnement. Les figures montrent une membrane déformable réalisée sous la forme d'une poche ou d'un diaphragme, mais ladite membrane pourrait être réalisée différemment, par exemple sous la forme d'un soufflet. Bien entendu, d'autres formes sont aussi envisageables.

La valve 200 n'est donc actionnée qu'au moment de l'inhalation, de sorte que la dose de produit fluide est expulsée hors de l'orifice de distribution simultanément à l'inhalation.

Lorsque l'utilisateur souhaite utiliser le dispositif, il place l'embout buccal 400 dans sa bouche, et appuie manuellement sur l'actionneur 20. Celui-ci peut alors coulisser axialement autour du réservoir 100 entre ses positions de repos et chargée pour charger le ressort 21, jusqu'à ce que l'actionneur 20 vienne en butée avec le corps 10, comme visible sur la figure 2. Le ressort comprimé 21 exerce une force axiale sur le réservoir 100 via ladite paroi proximale 22, mis ledit réservoir 100 est bloqué et empêché de coulisser dans le corps 10 par les pattes de blocage 501 de l'élément de blocage 500, qui s'appuient sur l'épaulement 710 du puits de soupape 700. Eventuellement, le réservoir 100 peut effectuer une petite course axiale initiale avant d'être bloqué, cette petite course initiale étant toutefois insuffisante pour actionner la valve doseuse 200. La figure 1 montre le dispositif au repos et la figure 2 montre le dispositif en position de blocage du réservoir 100, après chargement de l'actionneur 20 et avant inhalation.

Lorsque l'utilisateur inhale à travers l'embout buccal 400, il va déformer l'organe sensible à l'inhalation 60, ce qui va faire coulisser l'élément déclencheur 600 fixé audit organe sensible à l'inhalation 60, comme visible sur la figure 4. Ce déplacement de l'élément déclencheur 600 sur le puits de soupape 700 va libérer radialement les pattes 501 de l'élément de blocage 500. Sous l'effet de la force axiale transmise par le ressort 21 de l'actionneur 20 au réservoir 100, les pattes axiales 501 vont pouvoir se déformer radialement vers l'extérieur, et ainsi passer par-dessus ledit épaulement 710, pour permettre le coulissement du réservoir 100 vers sa position de distribution, et donc l'actionnement de la valve 200. Cette position de distribution est représentée sur la figure 6.

En fin d'inhalation, l'élément déclencheur 600 est ramené vers le haut par l'élasticité de la membrane de l'organe sensible à l'inhalation 60. Si nécessaire, l'élément déclencheur peut aussi être ramené manuellement en position de repos, en faisant coulisser le couvercle 650 axialement vers le haut.

Lorsque l'utilisateur relâche sa pression sur l'actionneur 20, le réservoir 100 revient en direction de la position de repos sous l'effet du ressort de rappel de la valve 200, et simultanément la soupape 210 de la valve doseuse revient en position de repos, en chargeant une nouvelle dose de produit fluide dans la chambre de valve. Parallèlement, l'actionneur 20 revient aussi en position de repos sous l'effet du ressort 21. Le dispositif est alors prêt pour une autre utilisation.

Il est à noter qu'avec l'actionneur 20, le dispositif peut aussi être actionné correctement si l'utilisateur inhale avant d'appuyer sur l'actionneur. Néanmoins, pour optimiser la synchronisation de la distribution de dose avec l'inhalation, il est préférable que l'actionneur 20 soit en position chargée au moment où l'utilisateur commence son inhalation.

La présente invention est notamment applicable pour le traitement des crises d'asthme ou de BPCO (Broncho Pneumopathie Obstructive) en utilisation avec des formulations de type salbutamol, aclidinium, formoterol, tiotropium, budesonide, fluticasone, indacaterol, glycopyrronium, salmeterol, umeclidinium bromide, vilanterol, olodaterol, striverdi, ou toute combinaison de ces formulations.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Dispositif de distribution de produit fluide synchronisé avec l'inhalation, comportant un corps (10) pourvu d'un embout buccal (400), un réservoir de produit (100) contenant un produit fluide et un gaz propulseur étant monté axialement coulissant dans ledit corps (10), une valve doseuse (200) comportant une soupape (210) étant assemblée sur ledit réservoir (100) pour distribuer sélectivement le produit fluide, ledit dispositif comportant:
- un élément de blocage (500) déplaçable et/ou déformable entre une position de non actionnement, dans laquelle ladite valve doseuse (200) ne peut pas être actionnée, et une position d'actionnement, dans laquelle ladite valve doseuse (200) peut être actionnée, ledit élément de blocage (500), en position de non actionnement, coopérant d'une part avec le corps (10) et d'autre part avec le réservoir (100) pour empêcher le déplacement axial dudit réservoir (100) dans le corps (10), et
- un système de déclenchement commandé par l'inhalation comportant un organe sensible à l'inhalation (60) déformable et/ou déplaçable sous l'effet de l'inhalation, ledit organe sensible à l'inhalation (60), lorsqu'il se déforme et/ou se déplace, déplaçant et/ou déformant ledit élément de blocage (500) de sa position de non actionnement vers sa position d'actionnement,
ledit dispositif comportant un actionneur (20) fixé sur ledit réservoir (100), ledit actionneur (20) comportant une paroi proximale (22) fixée, notamment emmanché à force, sur le fond du réservoir (100), un corps (23) coulissant axialement autour de ladite paroi proximale (22), et un ressort (21) disposé entre ladite paroi proximale (22) et une paroi distale (24) dudit corps (23), ledit corps (23) coulissant axialement autour de ladite paroi proximale (22) entre une position de repos et une position chargée, dans laquelle ledit ressort (21) est comprimé, de sorte qu'après actionnement dudit système de déclenchement commandé par l'inhalation, ledit ressort (21) comprimé déplace axialement ledit réservoir (100) dans ledit corps (10) pour actionner ladite valve doseuse (200).

2. Dispositif selon la revendication 1, dans lequel ledit élément de blocage (500) est une bague de blocage fixée, notamment encliquetée, sur ledit réservoir (100) et comportant au moins une patte axiale (501), notamment trois, coopérant avec un épaulement (710) solidaire dudit corps (10) pour bloquer le déplacement axial dudit réservoir (100) dans ledit corps (10).

3. Dispositif selon la revendication 2, dans lequel ladite au moins une patte axiale (501) est déformable radialement vers l'extérieur pour passer de la position de non actionnement vers la position d'actionnement, un élément déclencheur (600) étant prévu pour maintenir ladite au moins une patte axiale (501) en position de non actionnement.

4. Dispositif selon la revendication 3, dans lequel ledit élément déclencheur (600) est monté déplaçable entre une position de blocage, dans laquelle il bloque ledit élément de blocage (500) dans sa position de non actionnement, et une position de libération, dans laquelle il ne bloque pas ledit élément de blocage (500).

5. Dispositif selon la revendication 4, dans lequel ledit organe sensible à l'inhalation (60) dudit système de déclenchement commandé par l'inhalation comporte une membrane déformable définissant une chambre d'air déformable, ladite membrane déformable étant fixée audit élément déclencheur (600), ladite membrane déformable étant déformée lors de l'inhalation de telle sorte qu'elle déplace ledit élément déclencheur (600) de sa position de blocage vers sa position de libération.

6. Dispositif selon l'une quelconque des revendications précédentes, dans lequel ledit ressort (21) est un ressort à boudin.

7. Dispositif selon l'une quelconque des revendications 1 à 5, dans lequel ledit ressort (21) est un ressort plastique.

8. Dispositif selon l'une quelconque des revendications 1 à 5, dans lequel ledit ressort (21) est un élément en mousse.

9. Dispositif selon l'une quelconque des revendications précédentes, dans lequel ledit l'actionneur (20) comporte une bride radiale (25) s'étendant radialement vers l'extérieur de part et d'autre dudit corps (23).

10. Dispositif selon l'une quelconque des revendications précédentes, dans lequel la position de repos dudit actionneur (20) est définie par une butée axiale formée entre un bord axial inférieur (29) de ladite paroi proximale (22) et un épaulement radial interne (26) dudit corps (23).

11. Dispositif selon l'une quelconque des revendications précédentes, dans lequel la position chargée dudit actionneur (20) est définie par une butée axiale formée entre un bord axial inférieur (27) dudit corps (23) et un bord axial supérieur (801) d'un manchon interne (800) solidaire dudit corps (10).

## Patentansprüche

1. Vorrichtung zur inhalationssynchronisierten Abgabe eines Flüssigprodukts, wobei die Vorrichtung einen mit einem Mundstück (400) versehenen Körper (10) umfasst, wobei ein Produktvorratsbehälter (100), der ein Flüssigprodukt und ein Treibgas enthält, axial gleitend in dem Körper (10) angebracht ist, wobei zur selektiven Abgabe des Flüssigprodukts ein Dosierventil (200) mit einem Ventil (210) an dem Behälter (100) angebracht ist, wobei die Vorrichtung folgendes umfasst:
- ein Sperrelement (500), das zwischen einer Nichtbetätigungsstellung, in der das Dosierventil (200) nicht betätigt werden kann, und einer Betätigungsstellung, in der das Dosierventil (200) betätigt werden kann, verschoben und/oder verformt werden kann, wobei das Sperr-element (500) in der Nichtbetätigungsstellung einerseits mit dem Körper (10) und andererseits mit dem Vorratsbehälter (100) zusammenwirkt, um die axiale Bewegung des Vorratsbehälters (100) im Körper (10) zu verhindern, und
- ein inhalationsgesteuertes Auslösesystem, das ein auf die Inhalation ansprechendes Element (60) umfasst, welches durch die Inhalation verformbar und/oder verschiebbar ist, wobei das auf die Inhalation ansprechende Element (60) bei seiner Verformung und/oder Verschiebung das Sperrelement (500) aus dessen Nichtbetätigungsstellung in dessen Betätigungsstellung verschiebt und/oder verformt,
wobei die Vorrichtung folgendes umfasst: ein Stellglied (20), das an dem Behälter (100) befestigt ist, wobei das Stellglied (20) eine am Boden des Behälters (100) befestigte, insbesondere in diesen eingepresste proximale Wand (22), einen axial um die proximale Wand (22) herum gleitenden Körper (23), und eine zwischen der proximalen Wand (22) und einer distalen Wand (24) des Körpers (23) angeordnete Feder (21) umfasst, wobei der Körper (23) zwischen einer Ruhestellung und einer geladenen Stellung, in der die Feder (21) zusammengedrückt ist, axial um die proximale Wand (22) herum verschiebbar ist, sodass bei Betätigung des inhalationsgesteuerten Auslösesystems die zusammengedrückte Feder (21) eine axiale Bewegung des Vorratsbehälters (100) im Körper (10) bewirkt, um dadurch eine Betätigung des Dosierventils (200) zu erzielen.

2. Vorrichtung nach Anspruch 1, bei der das Sperrelement (500) ein Sicherungsring ist, der an dem Behälter (100) befestigt, insbesondere mit diesem verrastet, ist und mindestens eine axiale Lasche (501), insbesondere drei, aufweist, die mit einer mit dem Körper (10) fest verbundenen Schulter (710) zusammenwirkt, um die axiale Verschiebung des Vorratsbehälters (100) im Körper (10) zu blockieren.

3. Vorrichtung nach Anspruch 2, bei der die mindestens eine axiale Lasche (501) radial nach außen verformbar ist, sodass sie von der Nichtbetätigungsstellung in die Betätigungsstellung bewegt wird, wobei ein Auslöseelement (600) vorgesehen ist, um die mindestens eine axiale Lasche (501) in der Nichtbetätigungsstellung zu halten.

4. Vorrichtung nach Anspruch 3, bei der das Auslöseelement (600) zwischen einer Sperrstellung, in der es das Sperrelement (500) in seiner Nichtbetätigungsstellung verriegelt, und einer Freigabestellung, in der es das Sperrelement (500) nicht verriegelt, verschiebbar gelagert ist.

5. Vorrichtung nach Anspruch 4, bei der das auf Inhalation ansprechende Element (60) des inhalationsgesteuerten Auslösesystems eine verformbare Membran umfasst, die eine verformbare Luftkammer definiert, wobei die verformbare Membran an dem Auslöseelement (600) angebracht ist, wobei die verformbare Membran bei der Inhalation derart verformt wird, dass sie das Auslöseelement (600) aus seiner Sperrstellung in seine Freigabestellung bewegt.

6. Vorrichtung nach einem der vorhergehenden Ansprüche, bei der die Feder (21) eine Schraubenfeder ist.

7. Vorrichtung nach einem der Ansprüche 1 bis 5, bei der die Feder (21) eine Kunststofffeder ist.

8. Vorrichtung nach einem der Ansprüche 1 bis 5, bei der die Feder (21) ein Schaumstoffteil ist.

9. Vorrichtung nach einem der vorhergehenden Ansprüche, bei der das Stellglied (20) einen radialen Flansch (25) aufweist, der sich beidseitig des Körpers (23) radial nach außen erstreckt.

10. Vorrichtung nach einem der vorhergehenden Ansprüche, bei der die Ruhestellung des Stellglieds (20) durch einen axialen Anschlag definiert ist, der zwischen einem unteren axialen Rand (29) der proximalen Wand (22) und einer inneren radialen Schulter (26) des Körpers (23) gebildet ist.

11. Vorrichtung nach einem der vorhergehenden Ansprüche, bei der die geladene Stellung des Stellglieds (20) durch einen axialen Anschlag definiert ist, der zwischen einem unteren axialen Rand (27) des Körpers (23) und einem oberen axialen Rand (801) einer fest mit dem Körper (10) verbundenen inneren Hülse (800) gebildet ist.

## Claims

1. An inhalation-synchronized fluid dispenser device, comprising a body (10) provided with a mouthpiece (400), a fluid reservoir (100) containing a fluid and a propellant gas being mounted to slide axially in said body (10), a metering valve (200) including a valve member (210) being assembled on said reservoir (100) for selectively dispensing the fluid, said device further comprising:
• a blocking element (500) that is movable and/or deformable between a non-actuation position in which said metering valve (200) cannot be actuated, and an actuation position in which said metering valve (200) can be actuated, said blocking element (500), in its non-actuation position, co-operating firstly with the body (10) and secondly with the reservoir (100) so as to prevent said reservoir (100) from moving axially in the body (10), and
• an inhalation-controlled trigger system including an inhalation-sensitive member (60) that is deformable and/or movable under the effect of inhaling, said inhalation-sensitive member (60), when it is deformed and/or moved, moving and/or deforming said blocking element (500) from its non-actuation position towards its actuation position,
said device including an actuator (20) that is fastened on said reservoir (100), said actuator (20) comprising a proximal wall (22) that is fastened, in particular force-fitted, on the end of the reservoir (100), a body (23) that slides axially past said proximal wall (22), and a spring (21) that is arranged between said proximal wall (22) and a distal wall (24) of said body (23), said body (23) sliding axially past said proximal wall (22) between a rest position and a cocked position, in which said spring (21) is compressed, such that on said inhalation-controlled trigger system being actuated, said compressed spring (21) moves said reservoir (100) axially in said body (10) so as to actuate said metering valve (200).

2. A device according to claim 1, wherein said blocking element (500) is a blocking ring that is fastened, in particular snap-fastened, on said reservoir (100), and that includes at least one axial tab (501), in particular three axial tabs, co-operating with a shoulder (710) that is secured to said body (10) so as to prevent said reservoir (100) from moving axially in said body (10).

3. A device according to claim 2, wherein said at least one axial tab (501) is deformable radially outwards so as to pass from the non-actuation position towards the actuation position, a trigger element (600) being provided so as to hold said at least one axial tab (501) in the non-actuation position.

4. A device according to claim 3, wherein said trigger element (600) is mounted to move between a blocking position in which it blocks said blocking element (500) in its non-actuation position, and a release position, in which it does not block said blocking element (500).

5. A device according to claim 4, wherein said inhalation-sensitive member (60) of said inhalation-controlled trigger system includes a deformable membrane that defines a deformable air chamber, said deformable membrane being fastened to said trigger element (600), said deformable membrane being deformed during inhaling, so that it moves said trigger element (600) from its blocking position towards its release position.

6. A device according to any preceding claim, wherein said spring (21) is a coil spring.

7. A device according to any one of claims 1 to 5, wherein said spring (21) is a plastics spring.

8. A device according to any one of claims 1 to 5, wherein said spring (21) is a foam element.

9. A device according to any preceding claim, wherein said the actuator (20) includes a radial flange (25) that extends radially outwards on either side of said body (23).

10. A device according to any preceding claim, wherein the rest position of said actuator (20) is defined by an axial abutment that is formed between a bottom axial edge (29) of said proximal wall (22) and an inner radial shoulder (26) of said body (23).

11. A device according to any preceding claim, wherein the cocked position of said actuator (20) is defined by an axial abutment that is formed between a bottom axial edge (27) of said body (23) and a top axial edge (801) of an inner sleeve (800) that is secured to said body (10).
